# EUROPEAN PATENT APPLICATION

(11) **EP 0 930 313 A1**
(43) Date of publication of application: **21.07.1999**
(21) Application number: 99100069.6
(22) Date of filing: 05.01.1999
(51) Int. Cl.: C07D 417/04

(54) **1-isothiazolidinone derivatives of azetidine-2-one, process for the preperation thereof and the use thereof**

(30) Priority: 20.01.1998 HR 980026
(71) Applicant: PLIVA farmaceutska, kemijska, prehrambena i kozmeticka industrija, dionicko drustvo, 10000 Zagreb (HR)
(72) Inventor: Herak, Jure J., 10000 Zagreb (HR)
(74) Representative: von Füner, Alexander, Prof.h.c. Dr.

(57) **Abstract**

The object of the present invention are novel compounds of azetidine-2-one substituted in 1-position with different isothiazolidinone radicals, as analogons of a beta-lactam structure, of the general formula I 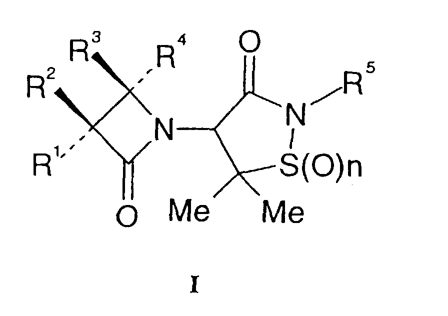 wherein
R¹ represents a hydrogen or a halo atom,
R² represents a hydrogen or a halo atom or a phthalimide group,
R³ represents a hydrogen or a halo atom,
R⁴ represents a hydrogen or a halo atom,
R⁵ represents a hydrogen atom, a lower linear or branched alkyl, aryl or aralkyl, or a radical containing a heterocycle with one or more heteroatoms, such as isoxazolyl or pyrazolyl, and
n is 0, 1 or 2.

The usefulness of these compounds as intermediates for the preparation of novel beta-lactam analogons or active substances in formulations for antimicrobial therapy is given.

## Description

### Technical Field

### IPC: C07D 513/04

The invention relates to novel isothiazolidinone derivatives of azetidine-2-one, processes for the preparation thereof and use thereof. These compounds are novel beta-lactam analogons constituted of a beta-lactam ring substituted in 1-position with various isothiazolidinone radicals, and as such represent stable intermediates for further chemical transformations to novel biologically active beta-lactam compounds.

According to our knowledge and available Prior Art literature data, neither said isothiazolidinone derivatives of azetidine-2-one nor processes for the preparation thereof have hitherto been known.

Well-known are numerous azetidine-2-one derivatives substituted in 1-position with differently functionalized linear or branched aliphatic chain, prepared by chemical transformations of penicillin derivatives or wholly synthetically.

So are numerous azetidine-2-ones having a substituent in 1-position in the form of unsubstituted or substituted cycloalkyl or aromatic rings, which are prepared synthetically.

The object of the present invention are 1-isothiazolidinone derivatives of azetidine-2-one of the general formula I 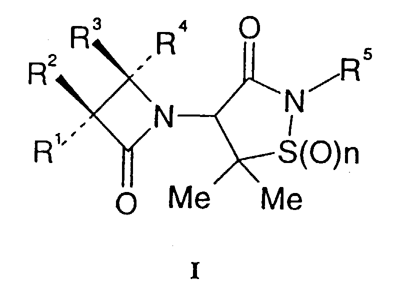 wherein
R¹ represents a hydrogen or a halo atom,
R² represents a hydrogen or a halo atom or a phthalimide group,
R³ represents a hydrogen or a halo atom,
R⁴ represents a hydrogen or a halo atom,
R⁵ represents a hydrogen atom, a lower linear or branched alkyl, aryl or aralkyl, or a radical containing a heterocycle having 5 or 6 members in the ring with one or more heteroatoms, the heteroatoms being oxygen, nitrogen or sulfur, such as isoxazolyl or pyrazolyl, and
n is 0, 1 or 2.

Another object of the present invention is a process for the preparation of 1-isothiazolidinone derivatives of azetidine-2-ones of the general formula I, wherein the radicals have the above meanings, which may be prepared by rearrangement of penicillanic acid sulfoxide amides of the general formula II 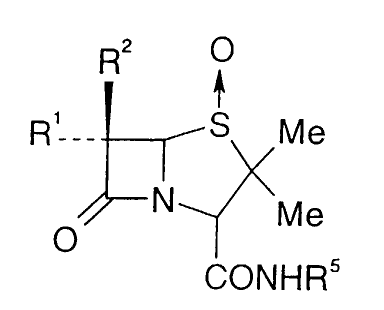 wherein
R¹ represents a hydrogen or a halo atom,
R² represents a hydrogen or a halo atom or a phthalimide group,
R⁵ represents a hydrogen atom, a lower linear or branched alkyl, aryl or aralkyl, or a radical contaninig a heterocycle having 5 or 6 members in a ring with one or more heteroatoms, the heteroatoms being oxygen, nitrogen or sulfur, such as isoxazolyl or pyrazolyl, and, optionally, by a subsequent oxidation of the obtained azetidine-2-ones of the general formula I, wherein the radicals have the above meanings and n is 0.

The process of the rearrangement of penicillanic acid sulfoxides amides of the general formula II is carried out in the presence of halogenating agents in anydrous inert organic solvents or without them at temperatures from -50°C to 150°C.

Suitable inert solvents in this reaction are e.g. anhydrous hydrocarbons such as benzene, toluene or xylene, ethers such as diethyl ether, dioxan or tetrahydrofuran, chlorinated hydrocarbons such as methylene chloride or chloroform, or carboxylic acid nitriles such as acetonitrile.

Suitable halogenating agents are thionyl chloride, thionyl bromide, sulfuryl chloride, hydrogen halides such as hydrogen chloride or hydrogen bromide.

The preparation of suitable starting compounds of the general formula II is an object of the Applicant's previous invention disclosed in HR patent application P940319A of 24 May 1994 with the title "Novel 2-Oxoazetidine Derivatives, Preparation Methods, Intermediates, Salts and Use", or is disclosed in a journal [*J. Chem. Research (S),* 176*;* (*M*) 1501 (1988)].

Their tendency to a rearrangement to novel bicyclic beta-lactam analogons was found when investigating Cooper rearrangement of penicillanic acid sulfoxides which do not possess an amide side chain in 6-position. The original Cooper rearrangement [*J. Am. Chem. Soc.,* **92**, 2575 (1972); Rearrangements of Cephalosporins and Penicillins, in *Cephalosporins and Penicillins*: eds. E. H. Flynn, Academic Press, New York, 1972, p. 201] is a well-known process for the preparation of thiazoline-azetidinone bicyclic structure by reductive rearrangement starting from penicillin G sulfoxides. Such a rearrangement requires the use of a protected carboxylic acid such as esters and a free carboxamide chain in 6-position as possessed by penicillins G and V.

It has been found earlier that starting from penicillanic acid sulfoxide amides of general formula II, i.e. such compounds of penam structure that have an amide group in 2-position but do not possess an amide side chain in 6-position, under similar conditions as are those of the Cooper rearrangement, there are formed compounds of penicillanic structure having an isopropylidene substituent in 2-position and an imino substituent in 3-position. This invention is also an object of the Applicant's earlier invention disclosed in HR patent application P 960131A of 21 March 1996, counterpart EP application 97104391.4, having the title "4-Thia-1-azabicyclo[3.2.0]heptane-3-imino-2-isopropylidene-7-oxo Analogons of Beta-lactams, Processes for Preparation and Use" or is disclosed in a journal [*Croat. Chem. Acta* **69** 1367-1376

(1996)]. Now it has been found that starting from penicillanic acid sulfoxides of the general formula II in the presence of chlorinating agents, there are formed compounds having a monocyclic beta-lactam structure of the general formula I, wherein
R¹ represents a hydrogen or a halo atom,
R² represents a hydrogen or a halo atom or a phthalimide group,
R³ represents a hydrogen or a halo atom,
R⁴ represents a hydrogen or a halo atom,
R⁵ represents a hydrogen atom, a lower linear or branched alkyl, aryl or aralkyl, or a radical containing a heterocycle having 5 or 6 members in a ring with one or more heteroatoms, the heteroatoms being oxygen, nitrogen or sulfur, such as isoxazolyl or pyrazolyl, and
n is 0.

All reactions of this step are carried out under usual reaction conditions and the obtained products are, after the treatment of the reaction mixture, isolated by crystallization or chromatography on a silica gel column.

Azetidine-2-one compounds of the general formula I, wherein the radicals have the above meanings and n is 0 may, depending on the strength of the oxidating agent, be optionally oxidized to corresponding sulfoxides or sulfones of the general formula I, wherein the radicals have the above meanings and n is 1 or 2. The oxidation may be carried out in an acidic or alkaline aqueous, organic or aqueous-organic solvent at a temperature from -50 to +100°C. Suitable oxidating agents are e.g. hydrogen peroxide, peracetic acid, *m*-chloroperbenzoic acid, potassium or sodium permanganate or potassium or sodium perchlorate.

All chemical reactions are carried out under usual reaction conditions and the obtained products are, after the treatment of the reaction mixture, isolated by crystallization or chromatography on a silica gel column.

A further object of the present invention relates to the use of the said compounds as useful reactants in the process of the preparation of novel monocyclic beta-lactams, potential intermediates for the preparation of beta-lactam antibiotics or synergists of beta-lactam antibiotics from the class of penems, oxipenems and carbapenems as well as to other possibilities offered by said substrates.

Still another object of the present invention relates to the use of the said compounds in combination with other beta-lactam antibiotics such as e.g. ampicillin, in ready-to-use drugs having a synergistic action.

The present invention is illustrated by the following Examples, which in no way should be construed as limitative.

### (3S,4R)-1-[(4S)-2-Benzyl-5,5-dimethyl-3-oxo-isothiazolidine-4-yl]-3-bromo-4-chloro-azetidine-2-one

### Example 1

To a solution of thionyl chloride (8 ml) in dry toluene (40 ml) (2*S*,4*S*,5*R*,6*S*)-2-benzyl-carbamoyl-6-bromo-3,3-dimethyl-7-oxo-4-thia-1-azabicyclo[3.2.0]heptane-4-oxide (1200 mg, 3.1 mmole) was added and it was stirred for 2 hours at room temperature. The reaction mixture was cooled to 5°C, a mixture of ice and water (40 g) was added and it was stirred at room temperature up to the dissolution of ice. The mixture was separated and the organic layer was washed with distilled water (3x30 ml), dried over Na₂SO₄ sicc., filtered and evaporated to dryness. A dry crude product (1017 mg) in the form of a foam was obtained. After chromatography on silica gel and elution with a toluene-ethyl acetate mixture, the foamy residue gave two substances. The substance with a R_{f} value 0.77 [toluene-ethyl acetate (3: 1, v/v)] was (3*S*,4*R*)-1-[(4*S*)-2-benzyl-5,5-dimethyl-3-oxo-isothiazolidine-4-yl]-3-bromo-4-chloro-azetidine-2-one.
Crystallization from diethyl ether gave a product in the form of a white solid, m.p. 107-108°C;
IR (KBr) νₘₐₓ/cm⁻¹: 1795vs, 1690vs, 1460m, 1350vs, 1220m, 1090m, 830s;
¹H NMR (300 MHz, CDCl₃) δ: 1.49 and 1.57 each (3H, s, CMe₂), 4.50 (1H, s, isot.-H), 4.52 and 4.67 each (1H, d, *J* = 10.6 Hz, CH₂), 4.93 (1H, s, 3-H), 6.30 (1H, s, 4-H), 7.26-7.36 (5H, m, C₆H₅);

| Anal. C₁₅H₁₆BrClN₂O₂S: | | | | |
|---|---|---|---|---|
| found: | C 44.45; | H 4.30; | N 7.25; | S 8.20%; |
| calc.: | C 44.62; | H 4.00; | N 6.94; | S 7.94%. |

### (3S,4S)-1-[(4S)-2-Benzyl-5,5-dimethyl-3-oxo-isothiazolidine-4-yl]-3-bromo-4-chloro-azetidine-2-one

### Example 2

The second substance obtained by chromatography of the residue after evaporation from Example 1 with R_{f} value 0.50 [toluene-ethyl acetate (3:1 v/v)] was (3*S*,4*S*)-1-[(4*S*)-2-benzyl-5,5-dimethyl-3-oxo-isothiazolidine-4-yl]-3-bromo-4-chloro-azetidine-2-one.
Crystallization from diethyl ether gave a product in the form of a white solid, m.p. 121-123°C;
IR (KBr) νₘₐₓ/cm⁻¹: 1796vs, 1685vs, 1455m, 1375m, 1338m, 1078m, 823s;
¹H NMR (300 MHz, CDCl₃) δ: 1.52 and 1.56 each (3H, s, CMe₂), 4.07 (1H, s, isot.-H), 4.48 and 4.72 each (1H, d, *J* = 14.1 Hz, CH₂), 5.40 (1H, d, *J* = 4.1 Hz, 3-H), 5.96 (1H, d, *J* = 4.1 Hz, 4-H), 7.26-7.36 (5H, m, C₆H₅);

| Anal. C₁₅H₁₆BrClN₂O₂S: | | | | |
|---|---|---|---|---|
| found: | C 44.85; | H 4.40; | N 6.75; | S 8.30%; |
| calc.: | C 44.62; | H 4.00; | N 6.94; | S 7.94%. |

### Example 3

Dry hydrogen chloride was blown for 15 minutes through a solution of (2*S*,4*S*,5*R*,6*S*)-2-benzyl-carbamoyl-6-bromo-3,3-dimethyl-7-oxo-4-thia-1-azabicyclo[3.2.0]heptane-4-oxide (1200 mg, 3.1 mmole) in dry toluene (50 ml). The reaction mixture was then stirred at room temperature for another hour, cooled to 5°C, a mixture of ice and water (40 g) was added and it was stirred at the same temperature up to the dissolution of ice. After treatment as in Examples 1 and 2, (3*S*,4*R*)-1-[(4*S*)-2-benzyl-5,5-dimethyl-3-oxo-isothiazolidine-4-yl]-3-bromo-4-chloro-azetidine-2-one and (3*S*,4*S*)-1-[(4*S*)-2-benzyl-5,5-dimethyl-3-oxo-isothiazolidine-4-yl]-3-bromo-4-chloro-azetidine-2-one were obtained.

### (4R)-1-[(4S)-2-Benzyl-5,5-dimethyl-3-oxo-isothiazolidine-4-yl]-3,3-dibromo-4-chloro-azetidine-2-one

### Example 4

To a solution of thionyl chloride (2 ml) in dry toluene (18 ml), (2*S*,4*R*,5*R*)-2-benzyl-carbamoyl-6,6-dibromo-3,3-dimethyl-7-oxo-4-thia-1-azabicyclo[3.2.0]heptane-4-oxide (300 mg, 0.65 mmole) was added at room temperature. The reaction mixture was heated to 80°C, stirred for further 2 hours at this temperature and cooled to 5°C. A mixture of ice and water (30 ml) was added and it was stirred up to the dissolution of ice. The reaction mixture was separated, the organic layer was washed with distilled water (3 x 30 ml), dried over Na₂SO₄ sicc., filtered and evaporated to dryness. A dry crude product (298 mg) in the form of a yellowish foam was obtained. After chromatography on silica gel with elution with methylene chloride, the foamy residue gave two substances. The substance with R_{f} value 0.86 [toluene-ethyl acetate (3:1 v/v)] was (4*R*)-1-[(4*S*)-2-benzyl-5,5-dimethyl-3-oxo-isothiazolidine-4-yl]-3,3-dibromo-4-chloro-azetidine-2-one and was obtained in the form of a white foam.
IR (film) νₘₐₓ/cm⁻¹: 1802vs, 1679vs, 1457m, 1375m, 1344vs, 1262m, 1112s, 812m;
¹H NMR (300 MHz, CDCl₃) δ: 1.47 and 1.57 each (3H, s, CMe₂), 4.59 (1H, s, isot.-H), 4.52 and 4.65 each (1H, d, *J* = 14.6 Hz, CH₂), 6.74 (1H, s, 4-H), 7.26-7.36 (5H, m, C₆H₅);

| Anal. C₁₅H₁₅Br₂ClN₂O₂S: | | | | |
|---|---|---|---|---|
| found: | C 37.12; | H 3.00; | N 6.05; | S 6.94%; |
| calc.: | C 37.33; | H 3.13; | N 5.80; | S 6.64%. |

### (4S)-1-[(4S)-2-Benzyl-5,5-dimethyl-3-oxo-isothiazolidine-4-yl]-3,3-dibromo-4-chloro-azetidine-2-one

### Example 5

The second substance isolated by chromatography of the residue after evaporation from Example 3 with R_{f} value 0.77 [toluene-ethyl acetate (3:1 v/v)] was (4*S*)-1-[(4*S*)-2-benzyl-5,5-dimethyl-3-oxo-isothiazolidine-4-yl]-3,3-dibromo-4-chloro-azetidine-2-one and was obtained in the form of a foam.
IR (film) νₘₐₓ/cm⁻¹: 1805vs, 1689vs, 1457m, 1376m, 1336vs, 1262m, 1102s, 799m;
¹H NMR (300 MHz, CDCl₃) δ: 1.44 and 1.47 each (3H, s, CMe₂), 4.00 (1H, s, isot.-H), 4.43 and 4.64 each (1H, d, *J* = 14.6 Hz, CH₂), 6.04 (1H, s, 4-H), 7.26-7.29 (5H, m, C₆H₅);

| Anal. C₁₅H₁₅Br₂ClN₂O₂S: | | | | |
|---|---|---|---|---|
| found: | C 37.40; | H 3.30; | N 5.85; | S 6.54%; |
| calc.: | C 37.33; | H 3.13; | N 5.80; | S 6.64%. |

### (3S,4R)-1-[(4S)-2-Benzyl-5,5-dimethyl-1,3-dioxo-isothiazolidine-4-yl]-3-bromo-4-chloro-azetidine-2-one

### Example 6

A solution of (3*S*,4*R*)-1-[(4*S*)-2-benzyl-5,5-dimethyl-3-oxo-isothiazolidine-4-yl]-3-bromo-4-chloro-azetidine-2-one (200 mg, 0.50 mmole) and *m*-chloroperbenzoic acid (85%, 100 mg, 0.6 mmole) in methylene chloride (10 ml) was stirred at room temperature for 1 hour. The reaction mixture was then washed with a 5% aqueous NaHCO₃ solution (20 ml) and distilled water (20 ml). The organic extract was dried over Na₂SO₄ sicc., filtered and evaporated to dryness. A dry crude product (180 mg) in the form of a yellowish foam was obtained. After crystallization from diethyl ether the foamy residue gave a crystalline product with R_{f} value 0.57 [toluene-ethyl acetate (3:1 v/v)] and m.p. 118-120°C, identified as (3*S*,4*R*)- 1-[(4*S*)-2-benzyl-5,5-dimethyl-1,3-dioxo-isothiazolidine-4-yl]-3-bromo-4-chloro-azetidine-2-one, in the form of a white foam.
IR (film) νₘₐₓ/cm⁻¹: 1788vs, 1724vs, 1452m, 1400m, 1324s, 1259m, 1110s, 790m;
¹H NMR (300 MHz, CDCl₃) δ: 1.25 and 1.57 each (3H, s, CMe₂), 4.66 (1H, s, isot.-H), 4.58 and 5.09 each (1H, 2d, *J* = 15.2 Hz, CH₂), 4.97 (1H, s, 3-H), 6.03 (1H, s, 4-H), 7.26-7.36 (5H, m, C₆H₅);

| Anal. C₁₅H₁₆BrClN₂O₃S: | | | | |
|---|---|---|---|---|
| found: | C 42.76; | H 3.60; | N 6.35; | S 8.00%; |
| calc.: | C 42.92; | H 3.84; | N 6.67; | S 7.64%. |

### (4R)-1-[(4S)-2-Benzyl-5,5-dimethyl-1,3-dioxo-isothiazolidine-4-yl]-3,3-dibromo-4-chloro-azetidine-2-one

### Example 7

By the action of *m*-chloroperbenzoic acid upon (4*R*)-1-[(4*S*)-2-benzyl-5,5-dimethyl-3-oxo-isothiazolidine-4-yl]-3,3-dibromo-4-chloro-azetidine-2-one in methylene chloride as in Example 6 and after chromatography on a silica gel column with eluting with a toluene-ethyl acetate mixture (4*R*)-1-[(4*S*)-2-benzyl-5,5-dimethyl-1,3-dioxo-isothiazolidine-4-yl]-3,3-dibromo-4-chloro-azetidine-2-one (90%) in the form of a white foam was obtained; R_{f} value 0.66 [toluene-ethyl acetate (3:1)].
IR (film) νₘₐₓ/cm⁻¹: 1806vs, 1724vs, 1457m, 1375m, 1342s, 1314s, 1265m, 1108vs, 799m;
¹H NMR (300 MHz, CDCl₃) δ: 1.23 and 1.60 each (3H, s, CMe₂), 4.59 and 5.09 each (1H, d, *J* = 15.3 Hz, CH₂), 4.78 (1H, s, isot.-H), 6.51 (1H, s, 4-H), 7.26-7.35 (5H, m, C₆H₅);

| Anal. C₁₅H₁₅Br₂ClN₂O₃S: | | | | |
|---|---|---|---|---|
| found: | C 36.36; | H 3.25; | N 5.40; | S 6.25%; |
| calc.: | C 36.12; | H 3.03; | N 5.62; | S 6.43%. |

### (4S)-1-[(4S)-2-Benzyl-5,5-dimethyl-1,3-dioxo-isothiazolidine-4-yl]-3,3-dibromo-4-chloro-azetidine-2-one

### Example 8

By the action of *m*-chloroperbenzoic acid upon (4*S*)-1-[(4*S*)-2-benzyl-5,5-dimethyl-3-oxo-isothiazolidine-4-yl]-3,3-dibromo-4-chloro-azetidine-2-one in methylene chloride as in Example 6 and after chromatography on a silica gel column with eluting with a toluene-ethyl acetate mixture (4*S*)-1-[(4*S*)-2-benzyl-5,5-dimethyl-1,3-dioxo-isothiazolidine-4-yl]-3,3-dibromo-4-chloro-azetidine-2-one (92%) in the form of a white foam was obtained; R_{f} value 0.66 [toluene-ethyl acetate (3:1)].
IR (film) νₘₐₓ/cm⁻¹: 1809vs, 1728vs, 1457m, 1375m, 1354s, 1265m, 1100vs, 788m;
¹H NMR (300 MHz, CDCl₃) δ: 1.29 and 1.55 each (3H, s, CMe₂), 4.31 (1H, s, isot.-H), 4.60 and 5.10 each (1H, 2d, *J* = 15.0 Hz, CH₂), 6.07 (1H, s, 4-H), 7.18-7.43 (5H, m, C₆H₅);

| Anal. C₁₅H₁₅Br₂ClN₂O₃S: | | | | |
|---|---|---|---|---|
| found: | C 36.00; | H 2.85; | N 5.75; | S 6.20%; |
| calc.: | C 36.12; | H 3.03; | N 5.62; | S 6.43%. |

### 4R and 4S isomers of 1-[(4S)-2-benzyl-5,5-dimethyl-1,1,3-trioxo-isothiazolidine-4-yl]-3,3-dibromo-4-chloro-azetidine-2-one

### Example 9

A solution of (4*R*)-1-[(4*S*)-2-benzyl-5,5-dimethyl-3-oxo-isothiazolidine-4-yl]-3,3-dibromo-4-chloro-azetidine-2-one (49 mg, 0.1 mmole) and *m*-chloroperbenzoic acid (85%, 60 mg. 0.3 mmole) in methylene chloride (10 ml) was stirred at room temperature for 3 hours and then at reflux for further 2 hours. The cooled reaction mixture was treated as in Example 6. A dry crude product (45 mg) was obtained in the form of a yellowish foam. After chromatography on a silica gel column with eluting with a toluene-ethyl acetate mixture, the foamy residue gave a product with an R_{f} value 0.35 (methylene chloride) and was identified as a mixture of 4*R* and 4*S* isomers of 1-[(4*S*)-2-benzyl-5,5-dimethyl-1,1,3-trioxo-isothiazolidine-4-yl]-3,3-dibromo-4-chloro-azetidine-2-one in a 4.5:5.5 ratio in the form of a white foam.
IR (film) νₘₐₓ/cm⁻¹: 1809vs, 1736vs, 1458m, 1341vs, 1304s, 1295m, 1119s, 1138vs, 1103m, 1041m;

| Anal. C₁₅H₁₅Br₂ClN₂O₄S: | | | | |
|---|---|---|---|---|
| found: | C 35.44; | H 3.22; | N 5.36; | S 5.95%; |
| calc.: | C 35.06; | H 2.94; | N 5.44; | S 6.23%. |

(4*R*)-1-[(4*S*)-2-Benzyl-5,5-dimethyl-1,1,3-trioxo-isothiazolidine-4-yl]-3,3-dibromo-4-chloro-azetidine-2-one was characterized as follows:
¹H NMR (300 MHz, CDCl₃) δ: 1.49 and 1.73 each (3H, s, CMe₂), 4.70 (1H, s, isot.-H), 4.75 and 4.77 each (1H, s, CH₂), 6.47 (1H, s, 4-H), 7.37-7.42 (5H, m, C₆H₅).

(4*S*)-1-[(4*S*)-2-Benzyl-5,5-dimethyl-1,1,3-trioxo-isothiazolidine-4-yl]-3,3-dibromo-4-chloro-azetidine-2-one was characterized as follows:
¹H NMR (300 MHz, CDCl₃) δ: 1.55 and 1.64 each (3H, s, CMe₂), 4.30 (1H, s, isot.-H), 4.75 and 4.77 each (1H, s, CH₂), 6.03 (1H, s, 4-H), 7.37-7.42 (5H, m, C₆H₅).

### Example 10

To a cooled (0-5°C) solution of (4*R*)-1-[(4*S*)-2-benzyl-5,5-dimethyl-3-oxo-isothiazolidine-4-yl]-3,3-dibromo-4-chloro-azetidine-2-one (49 mg, 0.1 mmole) in ethyl acetate (5 ml) and acetic acid (1 ml), a saturated aqueous KMnO₄ solution was slowly added up to the maintaining of red colour. The reaction mixture was then decoloured by adding an aqueous solution of hydrogen peroxide and the layers were separated. The organic layer was treated as in Example 9 and the said 4*R* and 4*S* diastereoisomer mixture was obtained.

### Example 11

By the action of *m*-chloroperbenzoic acid upon (4*S*)-1-[(4*S*)-2-benzyl-5,5-dimethyl-3-oxo-isothiazolidine-4-yl]-3,3-dibromo-4-chloro-azetidine-2-one in methylene chloride as in Example 9 and after chromatography on a silica gel column with eluting with a toluene-ethyl acetate mixture, a mixture of (4*R*)-1-[(4*S*)-2-benzyl-5,5-dimethyl-1,1,3-trioxo-isothiazolidine-4-yl]-3,3-dibromo-4-chloro-azetidine-2-one and (4*S*)-1-[(4*S*)-2-benzyl-5,5-dimethyl-1,1,3-trioxo-isothiazolidine-4-yl]-3,3-dibromo-4-chloro-azetidine-2-one in approximately the same quantities was obtained.

### Example 12

By the action of *m*-chloroperbenzoic acid upon (4*R*)-1-[(4*S*)-2-benzyl-5,5-dimethyl-1,3-dioxo-isothiazolidine-4-yl]-3,3-dibromo-4-chloro-azetidine-2-one in methylene chloride as in Example 9 and after chromatography on a silica gel column with eluting with a toluene-ethyl acetate mixture, a mixture of (4*R*)-1-[(4*S*)-2-benzyl-5,5-dimethyl-1,1,3-trioxo-isothiazolidine-4-yl]-3,3-dibromo-4-chloro-azetidine-2-one and (4*S*)-1-[(4*S*)-2-benzyl-5,5-dimethyl-1,1,3-trioxo-isothiazolidine-4-yl]-3,3-dibromo-4-chloro-azetidine-2-one in approximately the same quantities was obtained.

### Example 13

By the action of *m*-chloroperbenzoic acid upon (4*S*)-1-[(4*S*)-2-benzyl-5,5-dimethyl-1,3-dioxo-isothiazolidine-4-yl]-3,3-dibromo-4-chloro-azetidine-2-one in methylene chloride as in Example 9 and after chromatography on a silica gel column with eluting with a toluene-ethyl acetate mixture, a mixture of (4*R*)-1-[(4*S*)-2-benzyl-5,5-dimethyl-1,1,3-trioxo-isothiazolidine-4-yl]-3,3-dibromo-4-chloro-azetidine-2-one and (4*S*)-1-[(4*S*)-2-benzyl-5,5-dimethyl-1,1,3-trioxo-isothiazolidine-4-yl]-3,3-dibromo-4-chloro-azetidine-2-one in approximately the same quantities was obtained.

## Claims

1. 1-Isothiazolidinone derivatives of azetidine-2-one of the general formula I 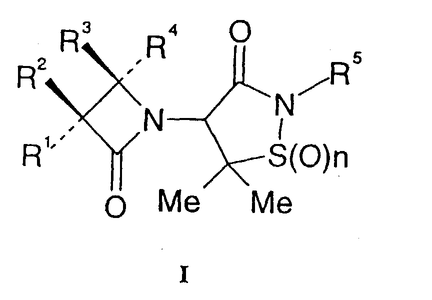 wherein
R¹ represents a hydrogen or a halo atom,
R² represents a hydrogen or a halo atom or a phthalimide group,
R³ represents a hydrogen or a halo atom,
R⁴ represents a hydrogen or a halo atom,
R⁵ represents a hydrogen atom, a lower linear or branched alkyl, aryl or aralkyl, or a radical containing a heterocycle having 5 or 6 members in the ring with one or more heteroatoms, the heteroatoms being oxygen, nitrogen or sulfur, such as isoxazolyl or pyrazolyl, and
n is 0, 1 or 2.

2. Compound according to claim 1, wherein R¹ is hydrogen, R² is hydrogen, R³ is hydrogen, R⁴ is chlorine, R⁵ is PhCH₂, and n is 0.

3. Compound according to claim 1, wherein R¹ is hydrogen, R² is hydrogen, R³ is chlorine, R⁴ is hydrogen, R⁵ is PhCH₂, and n is 0.

4. Compound according to claim 1, wherein R¹ is hydrogen, R² is bromine, R³ is chlorine, R⁴ is hydrogen, R⁵ is PhCH₂, and n is 0.

5. Compound according to claim 1, wherein R¹ is bromine, R² is hydrogen, R³ is chlorine, R⁴ is hydrogen, R⁵ is PhCH₂, and n is 0.

6. Compound according to claim 1, wherein R¹ is bromine, R² is hydrogen, R³ is chlorine, R⁴ is hydrogen, R⁵ is PhCH₂, and n is 1.

7. Compound according to claim 1, wherein R¹ is bromine, R² is hydrogen, R³ is chlorine, R⁴ is hydrogen, R⁵ is PhCH₂, and n is 2.

8. Compound according to claim 1, wherein R¹ is bromine, R² is bromine, R³ is chlorine, R⁴ is hydrogen, R⁵ is PhCH₂, and n is 0.

9. Compound according to claim 1, wherein R¹ is chlorine, R² is hydrogen, R³ is chlorine, R⁴ is hydrogen, R⁵ is PhCH₂, and n is 0.

10. Compound according to claim 1, wherein R¹ is bromine, R² is hydrogen, R³ is chlorine, R⁴ is hydrogen, R⁵ is 2-phenyl-pirazol-3-yl, and n is 0.

11. Compound according to claim 1, wherein R¹ is bromine, R² is hydrogen, R³ is chlorine, R⁴ is hydrogen, R⁵ is methyl, and n is 0.

12. Compound according to claim 1, wherein R¹ is hydrogen, R² is phthalimide, R³ is chlorine, R⁴ is hydrogen, R⁵ is PhCH₂, and n is 0.

13. Process for the preparation of 1-isothiazolidinone derivatives of azetidine-2-one of the general formula I, wherein the radicals have the meanings as given in claim 1, characterized in that penicillanic acid sulfoxides amides of the general formula II 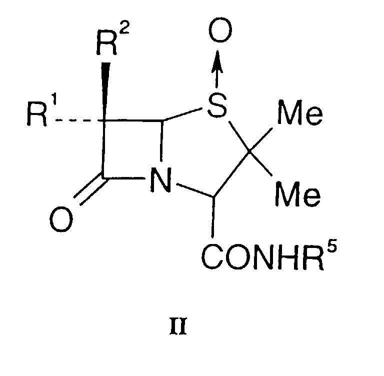 wherein
R¹ represents a hydrogen or a halo atom,
R² represents a hydrogen or a halo atom or a phthalimide group,
R⁵ represents a hydrogen atom, a lower linear or branched alkyl, aryl or aralkyl, or a radical containing a heterocycle having 5 or 6 members in the ring with one or more heteroatoms, the heteroatoms being oxygen, nitrogen or sulfur, such as isoxazolyl or pyrazolyl,
are subjected to reaction with halogenating agents such as thionyl chloride, thionyl bromide, sulfuryl chloride, hydrogen halides such as e.g. hydrogen chloride or hydrogen bromide, to achieve an opening of the thiazolidine ring of the compound II and then repeated intramolecular cyclization under formation of an isothiazolidinone ring and simultaneous halogenation of the azetidine ring, in a dry organic aprotic solvent under common reaction conditions, the reaction mixture is treated and products are isolated by generally known methods and, optionally, the obtained azetidine-2-one of the general formula I, wherein the radicals have previously given meanings and n is 0, is subsequently oxidized.

14. Use of the compounds according to claim 1 as novel beta-lactam analogons having a potential antibacterial action.
